# EUROPEAN PATENT APPLICATION

(11) **EP 4 154 823 A1**
(43) Date of publication of application: **29.03.2023**
(21) Application number: 21808362.4
(22) Date of filing: 13.05.2021
(51) Int. Cl.: A61B 10/00, A61B 13/00, A61B 5/08, A41D 13/11

(54) **SUBJECT SPECIMEN TOOL FOR VIRUS TESTING**

(30) Priority: 19.05.2020 KR 20200059759
(71) Applicant: Park, Yong Nam, Seoul 06068 (KR)
(72) Inventor: Park, Yong Nam, Seoul 06068 (KR)
(74) Representative: Rau, Schneck & Hübner Patentanwälte Rechtsanwälte PartGmbB
(86) International application number: PCT/KR2021/005989
(87) International publication number: WO 2021/235768

(57) **Abstract**

The present invention relates to a subject specimen tool for virus testing and, more specifically, to a subject specimen tool for virus testing, which enables an examiner to collect a specimen of a subject by using a specimen collection swab, while being worn by the subject, and is kept airtight during specimen collection, so that a contaminant in a mask does not leak outside of the mask, and thus, contamination around testing and infection of the examiner can be prevented. To this end, provided is a subject specimen tool for virus testing, comprising: a mask for shielding the face or a portion thereof of a subject; a specimen collection swab which is inserted into the mouth or nose of the subject wearing the mask, to collect a specimen; a swab insertion/withdrawal hole part formed in the mask and having a path with a diameter sufficient for the specimen collection swab to be inserted thereinto and withdrawn therefrom; and a detachable cap configured to be detachable from the swab insertion/withdrawal hole part and having an opening means which is open to guide the specimen collection swab to the path of the swab insertion/withdrawal hole part, wherein the opening means is open only in one direction toward the swab insertion/withdrawal hole part.

## Description

### Field of Invention

The present invention relates a subject specimen tool for virus test, and more particularly, to a subject specimen tool for virus inspection that can prevent a contaminant from leaking out of a mask worn by a subject while collecting a specimen for virus inspection in a worn state.

### Background of Invention

Masks have been used to cold protection effects by covering a persons' nose and mouth, or to provide dust protection for professionals who are exposed to specific harmful environments. Further, due to the influence of industrial development, the use of fossil fuels rapidly increases and the ozone layer is destroyed, which in turn increases UV index, therefore, people often use masks to block high index UV rays. In addition to the above uses, the mask is used for prevention of infectious diseases. In particular, recently, due to the pandemic of coronavirus infection-19 (COVID-19), a large number of infections and deaths are occurring around the world, therefore, it is essential to wear a mask in order to prevent infection and contagion.

Meanwhile, in the process of collecting specimens from suspected infections, a need for preventing contamination of the environment at the specimen collection site is emerging as an important issue so as to prevent contagion to the medical personnel who are examiners as well as the next test subject. For accurate virus inspection, it is important for medical personnel to collect a sufficient amount of specimens from the upper respiratory tract (oropharyngeal swab, nasopharyngeal swab, nasal swab, etc.). Such upper respiratory tract specimen collection is done by the examiner taking specimens from the oropharynx (mouth) and nasopharynx (nose) of the subject while taking off the mask and exposing the mouth and nose of the subject. At this time, when collecting the specimen from the mouse or nose of the subject, the medical personnel must use sterile cotton swabs and, even if the subject is somewhat uncomfortable, should swab the nasopharyngeal and palatopharyngeal walls several times in order to obtain sufficient specimen. During the specimen collection process, the subject is highly likely to sneeze or cough due to the stimulation of the reflex area. Therefore, due to such sneeze and cough of the subject, it is now facing problems of not only a broad range of space contamination such as droplet transmission and aerosol transmission in the specimen collection site but also very high possibility of infection and contagion to medical personnel. In fact, there are many cases of infection to the medical personnel who actually inspect suspected infections.

Accordingly, in the specimen collection process for the subject, the management of contaminants such as the discharge of droplets and aerosols due to the sneezing of the subject, the discharge of contaminants through respiration, etc., is emerging as a very significant problem.

### Prior Art Literature

### Patent Document

Korean Patent Laid-Open Publication No. 10-2012-0058658 Korean Registered Patent No. 10-1765474

### Summary of Invention

### Technical Problem to be Solved

The present invention has been devised to solve the above problems, and an object of the present invention is to provide a subject specimen tool for virus inspection, which enables a face of the subject to be kept airtight in a process of swabbing a specimen collection cotton swab and collecting a specimen from the nasal cavity and the mouth of the face of the subject, so as to prevent contamination of a specimen collection site and infection of an examiner during virus inspection.

### Technical Solution

In order to achieve the above object, the present invention provides a subject specimen tool for virus inspection, which includes: a mask for shielding the face or a part of the face of a subject; a swab entry hole part formed in the mask and having a passage with a diameter, through which a specimen collection (cotton) swab can enter and exist; and a detachable cap provided with an opening means for guiding the specimen collection swab into the passage of the swab entry hole part, characterized in that the opening means is opened only in one direction toward the swab entry hole part.

In this case, the swab entry hole part may be formed in a sight glass corresponding to the mouth/nose of the subject, and the sight glass is preferably made of a transparent material protruding from a body of the mask toward the front side.

Specifically, the swab entry hole part may protrude forward from the mask while being formed to gradually narrow in diameter toward the front side, and the detachable cap is preferably formed in a hollow shape that can be forcedly-fitted to the swab entry hole part.

Further, the swab entry hole part may protrude forward from the mask, wherein a tip of the swab entry hole part is larger than that the diameter of the swab entry hole part to thus form a step due to a difference in diameters, while the detachable cap preferably has a locking part configured to be hooked and coupled on the step.

Further, the opening means may be provided with a resin material having a restoration force and is preferably formed in a plurality of incisions that may be divided around the center of the detachable cap so that, when inserting the specimen collection swap, the incisions can be rolled into the detachable cap.

Further, in the body of the mask, a portion corresponding to the mouth of the subject may further be provided with a tongue pressing plate, wherein the tongue pressing plate is made of a flexible material and thus is preferably configured such that the examiner can push a tongue depresser and the tongue pressing plate together into the subject's mouth while placing the tongue depresser on the tongue pressing plate.

### Effect of Invention

The subject specimen tool for virus inspection according to the present invention may exhibit the following effects.

The subject specimen tool for virus inspection may include: a swab entry hole part formed on a region corresponding to the nose and mouth of a subject, through which a specimen collection swab can enter and exit; and a detachable cap capable of covering and shielding the swab entry hole part, which is configured to be detachable on the swab entry hole part, wherein an opening means for guiding insertion of the swab collection swab is provided on the detachable cap to be opened only in one direction. Accordingly, the present invention enables the face of the subject to be kept airtight during collection of a specimen, whereby it is possible to prevent contamination around a test site due to breathing and droplets of the subject, as well as infection of medical personnel as the examiner.

### Brief Description of Drawings

FIG. 1 illustrates a subject specimen tool for virus inspection according to a preferred embodiment of the present invention.
FIGS. 2a and 2b show appearance before and after wearing the subject specimen tool for virus inspection according to a preferred embodiment of the present invention.
FIGS. 3a and 3b illustrate a process of inserting a specimen collection swab into the mouth and nose of a subject after wearing the subject specimen tool for virus inspection according to a preferred embodiment of the present invention.
FIG. 4 shows the specimen collection swab used for the subject specimen tool for virus inspection according to a preferred embodiment of the present invention.
FIGS. 5a and 5b illustrate a process of removing a detachable cap of the subject specimen tool for virus inspection according to a preferred embodiment of the present invention by the specimen collection swab.
FIGS. 6 and 7 illustrates a modified example of a structure for mounting the detachable cap of the subject specimen tool for virus inspection according to a preferred embodiment of the present invention.
FIGS. 8a and 8b illustrate a subject specimen tool for virus inspection according to another embodiment of the present invention.
FIGS. 9a and 9b illustrate a subject specimen tool for virus inspection according to a further embodiment of the present invention.

### Best Mode for Carrying out Invention

The terms or words used in the present specification and claims are not to be construed as being limited to their ordinary or dictionary meanings, instead, on the principle that the inventor can appropriately define the concept of the term in order to best describe his invention, those should be interpreted as the meanings and concept consistent with the technical idea of the present invention.

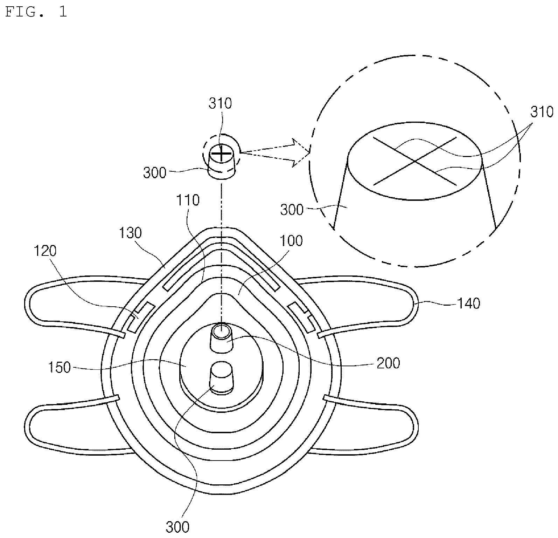

Hereinafter, a subject specimen tool for virus inspection (hereinafter referred to as a "specimen tool") according to a preferred embodiment of the present invention will be described with reference to the accompanying FIGS. 1 to 7. The specimen tool allows an examiner to swab and collect a specimen using a specimen collection (cotton) swab even when a subject is wearing the specimen tool, while minimizing the leakage of the subject's breathing or droplets so as to inhibit contamination of the surrounding environment and prevent infection of the examiner. The specimen tool, as shown in FIG. 1, may include a mask 100, a swab entry hole part 200, a detachable cap 300 and a specimen collection swab 400.

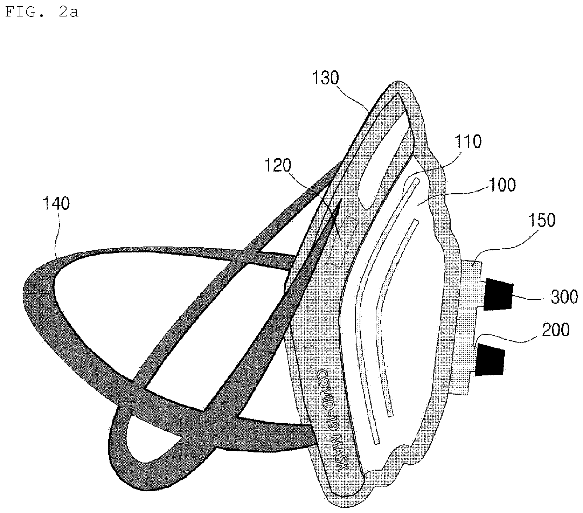

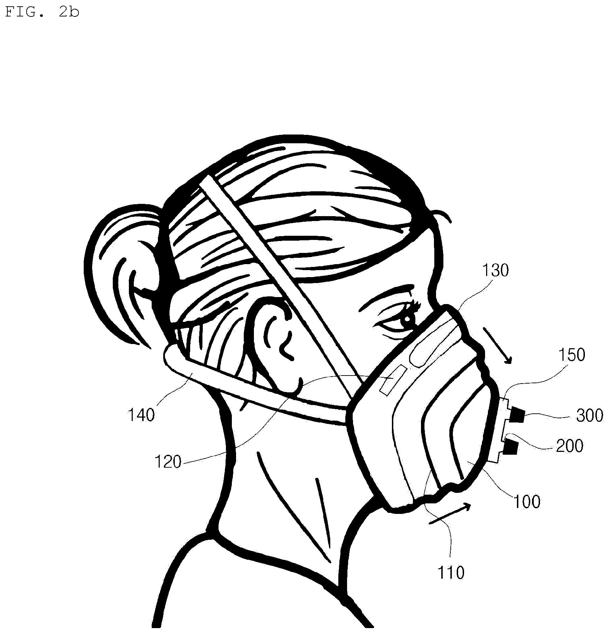

The mask 100 may form a part of the specimen tool, and may be configured to shield at least the nose and mouth of a subject. For convenience of explanation, the mask 100 is shown in a size to cover a person's nose and mouth, but the mask 100 may also be configured to shield the entire face of a person. The mask 100 is preferably provided using a transparent resin material. Since the mask 100 is used for preventing the wearer's breathing from leaking out rather than for the purpose of breathing, it is preferably made of a resin material. Of course, the material of the mask 100 is not limited to such resin material but may adopt various substances such as cloth material, urethane, and the like. The mask 100 is preferably provided to be stretchable through a wrinkle part 110 to secure a breathing space for the wearer and to flexibly respond to the wearer's face size. That is, as shown in FIG. 2a, the mask 100 is usually reduced in volume and, when worn, may be stretched through the wrinkle 110 so that the efficiency of storage or carrying and wearing the mask can be increased.

The mask 100 may have a breathing filter 120 mounted at the upper end of the mask 100 to facilitate the wearer's breathing even a little. Since breathing may be difficult due to characteristics of the material of the mask 100 when the wearer is an elderly person or an infant, the mask 100 may be provided with the breathing filter 120 to help the wearer breathe. Further, the breathing filter 120 may also serve to minimize the fogging on an inner surface of the mask 100 while wearing the mask. Of course, in addition to the above filter, anti-fogging of the mask 100 may also be achieved by application of anti-fogging coating to the inner surface of the mask 100. Further, a wire may be mounted on the upper end of the mask 100, that is, a portion corresponding to the wearer's nose in order to increase air-tightness. On the other hand, it is preferable that a face contact portion 130 is provided along the edge of the mask 100. The face contact portion 130 is a portion in contact with the wearer's face, and may be configured to improve the wearing comfort and air-tightness. The face contact portion 130 is preferably made of a flexible and stretchable material. Further, a wearing strap 140 may be provided on the mask 100 to facilitate wearing.

A sight glass 150 is preferably configured in the central portion of the mask 100. The sight glass 150 is a portion on which the swab entry hole part 200 is configured, and may have a configuration to secure a field of view when the examiner inserts the specimen collection swab 400 into the subject's mouth or nose. The sight glass 150 may have a flat front surface to prevent distortion, and may be formed of a transparent material. Although a shape of the sight glass 150 is not specified, a circular shape protruding from the mask 100 is preferably used as shown in FIG. 1.

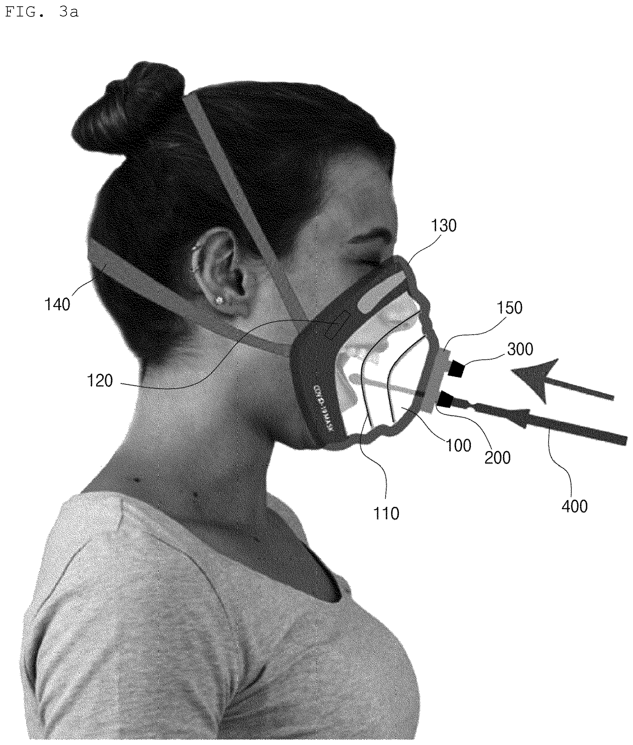

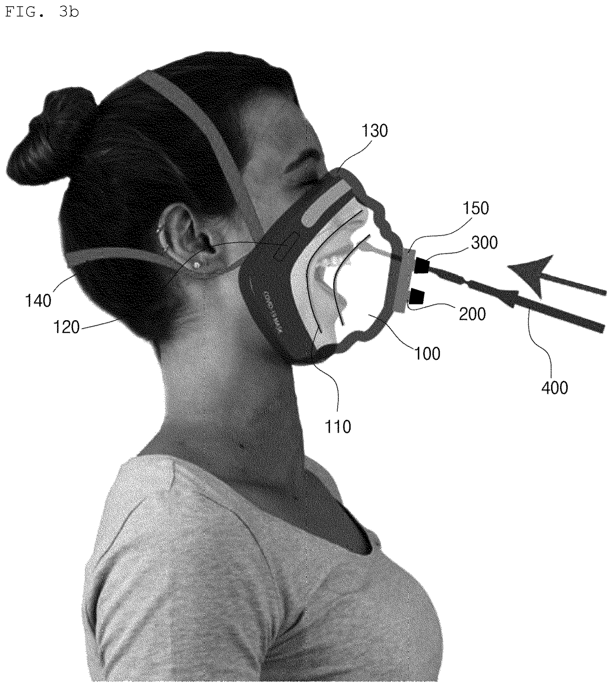

The swab entry hole part 200 may provide a passage through which the specimen collection swab enters and exits toward the wearer's respiratory tract, and may be formed in the sight glass 150. The number of the swab entry hole part 200 is one or more and, as shown in FIGS. 3a and 3, when considering that the specimen collection swabs 400 are typically inserted into the wearer's nose or mouth, two of the swab entry hole parts 200 are preferably used. The swab entry hole part 200 is opened and closed by the detachable cap 300 and is preferably formed to be inclined for fitting with the detachable cap 300. That is, the swab entry hole part 200 may be formed to protrude forward from the sight glass 150, wherein a diameter of the swab entry hole part 200 becomes narrower toward the front side. Such a configuration of the swab entry hole part 200 may be effective in forced-fitting the detachable cap 300.

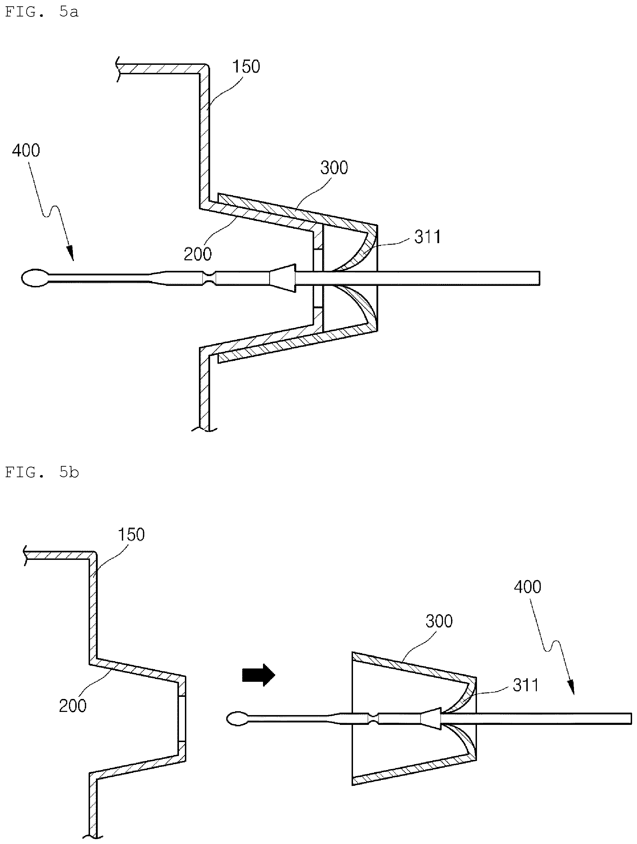

The detachable cap 300 may cover the swab entry hole part 200 in order to close the passage of the swab entry hole part 200. Further, this may be provided to be detachably attached to the swab entry hole part 200 and formed of a resin material. The detachable cap 300 is preferably configured to be forcedly fitted into the swab entry hole part 200 and is also preferably formed to have an inclined surface as shown in FIGS. 1 and 5a. The detachable cap 300 may have a hollow shape with one side opened, while being provided with an opening means 310 on the other side of the detachable cap 300. The opening means 310 may be a structure for guiding insertion of the specimen collection swab 400 into the swab entry hole part 200, and may be configured to be opened only in one direction toward the swab entry hole part 200. With this configuration, the detachable cap 300 may maintain air-tightness even when the specimen collection swab 400 is inserted into the detachable cap 300 through the opening means 310. The opening means 310 is not particularly limited in configuration but is preferably formed with an incision line 310 that can cut the other side of the detachable cap 300 in all directions as shown in FIG. 1. The incision line 310 may be provided as a line formed with a thinner thickness than that of the detachable cap 300 and thus is configured to be incised when an external force is applied to the specimen collection swab 400. Accordingly, the opening means 310 is incised toward the swab entry hole part 200 as shown in FIG. 5a at the moment when the specimen collection swab 400 passes through the hole, due to the material characteristics of the detachable cap 300, and may open in one direction to thus form an incision 311. Of course, the incision line 310 may be provided in an already cut state. Further, although not shown, the shape of the incision line 310 may be provided in various manners. For example, the incision line 310 is formed in a circular shape having only one fixed side and, when a force is applied to the specimen collection swab 400, the circular incision may be rolled into around the one side.

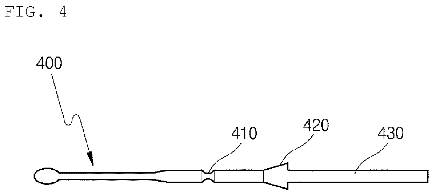

Meanwhile, the specimen collection swab 400 may be provided for collecting a specimen from the subject, and the specimen collection swab 400 may include a cut part 410, an interference plate 420 and a handle 430, as shown in FIG. 4. The cut part 410 is a configuration for separating the specimen collection swab 400 and the handle 430, and may be formed smaller than each diameter of the specimen collection swab 400 and the handle 430. The interference plate 420 is a configuration in that, when the specimen collection swab 400 swabs a specific body part of the subject through the swab entry hole part 200 and then comes out of the mask 100, the interference plate interferes with the detachable cap 300 so as to prevent the specimen from being in contact with the detachable cap 300, which in turn, removes the detachable cap 300 from the swab entry hole part 200. The interference plate 420 is formed to be larger than a diameter of the handle 430 but may have a smaller diameter than the diameter of the swab entry hole part 200. The handle 430 may be provided in a bar shape to be gripped by the examiner. On the other hand, although not shown, the interference plate 420 is composed of a wrinkle portion which may be provided as a set with the opening means for forming the above-described circular incision. That is, the specimen collection swab 400 may be provided such that it cuts and passes through the incision line 310 and, when the examiner pulls the specimen collection swab 400, interferes with the incision line 310 located in a groove of the wrinkle part, thereby removing the detachable cap 300.

Hereinafter, a process of collecting a specimen by the examiner after the subject wears a subject mask having the above configuration will be described.

The subject may wear the mask 100 on the face using the wearing strap 140. At this time, the face contact part 130 may be in close contact with the wearer's face to allow a breathing space (facial space) to be kept airtight.

Next, the examiner may insert the specimen collection swab 400 into the mouth or nose of the subject as shown in FIGS. 3a and 3b, followed by swabbing. To this end, the examiner pushes the specimen collection swab 400 into the opening means 310 of the detachable cap 300. At this time, the specimen collection swab 400 may be inserted while cutting and opening the incision line 310 as an opening means, as shown in FIG. 5a, and then, may enter the breathing space through a passage of the swab entry hole part 200. In this process, the opened incision 311 may be in close contact with the handle 430 of the specimen collection swab 400 while being restored. Accordingly, the breathing space of the subject may be kept airtight and, even if the subject sneezes and a pressure is generated in the breathing space, the incision 311 does not unfold outward, and therefore, no breathing or droplets leak out of the mask 100.

Thereafter, the examiner may check the subject's face through the sight glass 150 and the mask 100, and carry out swabbing with the specimen collection swab 400. When the swabbing is completed, the specimen collection swab 400 is removed from the mask 100.

When the examiner pulls the specimen collection swab 400 out of the mask 100, the specimen collection swab 400 moves to the right in the drawing, and the interference plate 420 interferes with the incision 311 to thus apply a removal pressure to the detachable cap 300. Thereafter, the specimen collection swab 400 may separate the detachable cap 300 from the swab entry hole part 200, as shown in FIG. 5b. Then, the examiner cuts the cut part 410 of the specimen collection swab 400 exposed to the outside, followed by conducting virus diagnosis through a test kit.

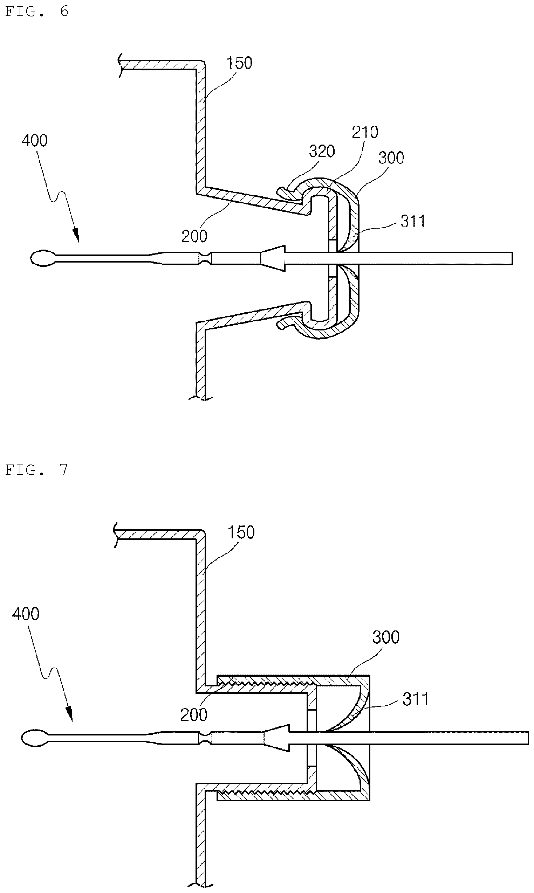

On the other hand, a coupling structure between the detachable cap 300 and the swab entry hole part 200 may be provided in various ways. As described above, the detachable cap 300 may be forcedly fitted to the swab entry hole part 200 through the inclined surface structure, or may be coupled as shown in FIG. 6. FIG. 6 illustrates a modified example of the coupling between the detachable cap 300 and the swab entry hole part 200, wherein a tip of the swab entry hole part 200 is formed to have a diameter larger than that of the swab entry hole part 200, thereby forming a step. Further, a locking part 320 may be formed in the detachable cap 300 and thus be coupled to the step 210 of the swab entry hole part 200. With such a configuration, the detachable cap 300 may be coupled to the swab entry hole part 200 since the locking part 320 is opened by the step 210 and then caught thereto to be supported while being restored. Thereafter, a series of processes for specimen collection after swabbing using the specimen collection swab 400 may be the same as the above-described processes, therefore, a detailed description thereof will be omitted.

FIG. 7 is another modified example of the coupling between the detachable cap 300 and the swab entry hole part 200, wherein the detachable cap 300 and the swab entry hole part are configured to be screw-coupled to each other. A female screw is formed on the inner circumferential surface of the detachable cap 300 while a male screw is formed on the outer circumferential surface of the swab entry hole part 200, so as to be screwed together.

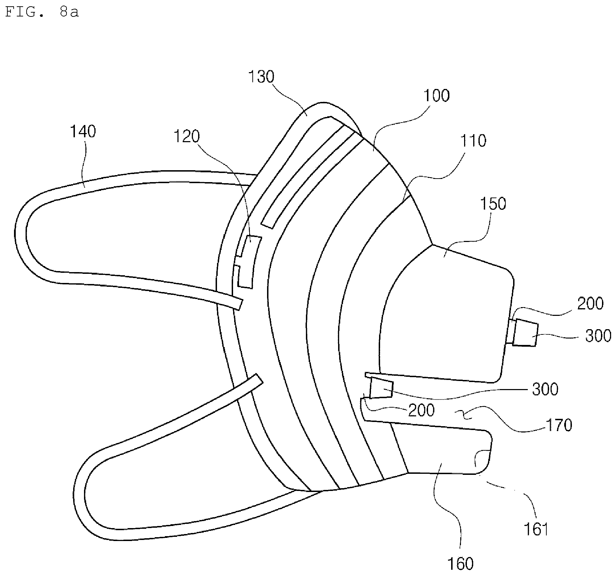

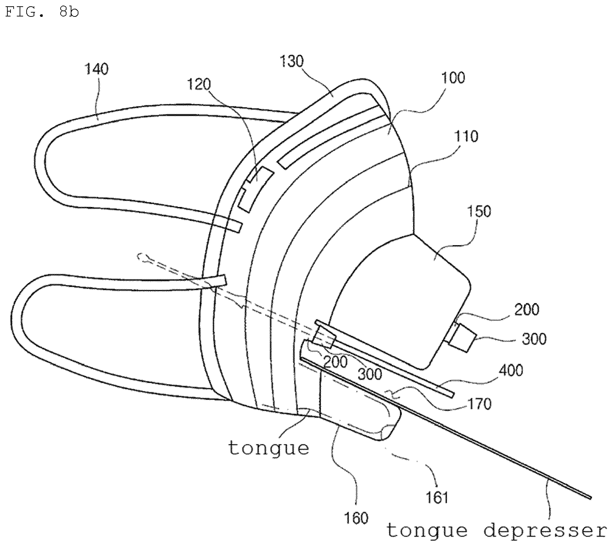

On the other hand, while the examiner collects a specimen from the subject's mouth, the tongue of the subject may unconsciously block the airway, making it difficult for the examiner to collect the specimen. Therefore, the specimen tool of the present invention may also be configured such that a tongue depresser can be introduced into the mouth while keeping airtight. This is presented as another embodiment of the present invention and will be described with reference to FIGS. 8a and 8b. Prior to description, the same reference numerals as in the preferred embodiment would be denoted by reference numerals.

As shown in FIG. 8a, the swab entry hole part 200 for swabbing the inside of the nose of a subject and the detachable cap 300 may be formed on the sight glass 150 of the mask 100, while the tongue pressing part 160 is provided at the lower part thereof. The tongue pressing part 160 may be mounted on a lower portion of the sight glass 150 to correspond to the mouth of the subject when wearing the mask 100. The tongue pressing part 160 may be formed at a position spaced downward from the sigh glass 150, on which the swab entry hole part 200 corresponding to the nose of the subject is provided, and may further include a groove 161 for receiving the tongue of the subject. Since the tongue pressing part 160 is spaced apart from the sight glass 150 provided with the swab entry hole part 200 corresponding to the nose, as shown in FIG. 8a, a guide groove 170 may also be formed between the tongue pressing part 160 and the sight glass 150. The guide groove 170 is a space to receive the tongue depresser of the examiner and, owing to the configuration of the guide groove 170, the tongue depresser may suppress the subject's tongue accommodated in the groove 161 of the tongue pressing part 160. At this time, the inside of the guide groove 170 may accommodate the swab entry hole part 200 for collecting a specimen in the mouth.

Then, the subject wears the mask 100 configured as described above, and the examiner presses the tongue pressing part 160 from above using the tongue depresser as shown in FIG 8b, thereby suppressing the tongue received in the groove 161. In this case, even if the examiner inserts the tongue depresser into the subject's mouth from the outside of the mask 100, the tongue depresser may indirectly suppress the subject's tongue through the tongue pressing part 160 so that a breathing space in the mask 100 can be kept airtight continuously. Thereafter, the examiner may push the specimen collection swab 400 into the detachable cap 300 located in the guide groove 170 in order to collect the specimen. As such, the examiner may use the tongue depresser so as to improve convenience in collecting the specimen in the mouth. Then, the examiner may pull the specimen collection swab 400 as well as the tongue depresser, out of the mask 100. At this time, the tongue pressing part 160 is restored and returned to its original position.

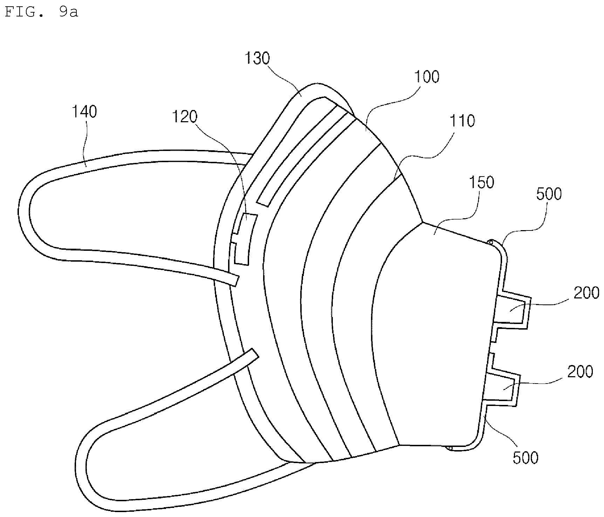

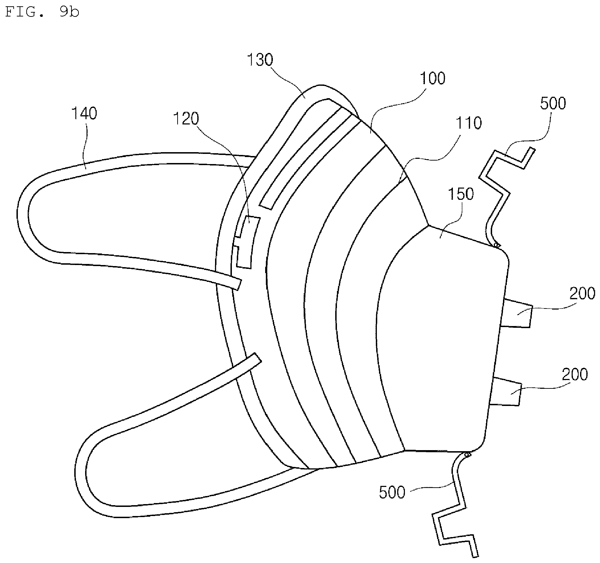

On the other hand, when the specimen collection for the subject is completed, it is understandable that the detachable cap 300 is separated and the swab entry hole part 200 becomes in an open state. At this time, it is concerned that the breath of the subject who has completed the specimen collection may leak through the swab entry hole part 200 and, when the subject sneezes, droplets may also leak out of the mask 100 through the swab entry hole part 200. Therefore, in order to prevent the contaminant from leaking out of the mask 100 of the subject after completing the specimen collection, a shielding cover 500 may be further provided. This is presented as another embodiment of the present invention and will be described with reference to the accompanying FIGS. 9a and 9b. Prior to the description, the same reference numerals as in the above-described embodiments would be denoted by reference numerals.

As shown in FIGS. 9a and 9b, the shielding cover 500 is provided to shield each of the swab entry hole parts 200. In the present specification, as two swab entry hole parts 200 are present, two of the shielding covers may also be provided. Herein, as shown in FIG. 9b, the shielding cover 500 on one side is axially coupled to the upper end of the sight glass 150 and rotates downward to open and close the swab entry hole part 200, while the shield cover 500 on the other side is axially coupled to the lower end of the sight glass 150 and rotates upward to open and close the swab entry hole part 200. With such a configuration, each shielding cover 500 may open and close the swabs entry hole part 200, from which the detachable cap 300 has been separated. At this time, although not shown, it is preferable that a fixing means may be mounted on each shielding cover 500. The fixing means is for restricting the movement of the shielding cover 500 when the shielding cover 500 closes the swab entry hole part 200. The fixing means is not limited to a specific construction and may be provided in various configurations such as hooks and buttons. On the other hand, although the shielding cover 500 is configured to be rotatable in the upper and lower directions as an example in the drawings, the present invention is not limited thereto but the shielding cover 500 may also be configured to be rotatable in the left and right directions of the mask 100.

With such a configuration, even when the inspection of the subject is completed, the mask 100 can shield the swab entry hole part 200 using the shielding cover 500. Therefore, it is possible not only to prevent the air in the mask from leaking out of the mask 100, but also to prevent droplets from leaking out of the mask 100 even when the subject sneezes, thereby fundamentally blocking the source of contamination. As described above, a subject specimen tool for virus inspection according to the present invention allows specimen collection to be done in even a state in which the subject wears the tool, while keeping airtight from the outside even if the specimen collection swab is inserted into the mask, so that it is possible to fundamentally block a source of contamination, thereby preventing contamination of the environment around a test site and infection of the examiner. Although the present invention has been described in detail with respect to the aforementioned embodiments, it is apparent to those skilled in the art that various modifications and variations are possible within the scope of the technical spirit of the present invention, and it is natural that such variations and modifications belong to the appended claims.

## Claims

1. A subject specimen tool for virus inspection, comprising:
a mask for shielding a face or a part of the face of a subject;
a specimen collection cotton swab which is inserted into a mouth or a nose of the subject wearing the mask to collect a specimen;
a swab entry hole part formed in the mask and having a passage with a diameter, through which the specimen collection cotton swab can enter and exit; and
a detachable cap which is configured to be detachable on the swab entry hole part and is provided with an opening means for guiding the specimen collection cotton swab into the passage of the swab entry hole part,
wherein the opening means is opened only in one direction toward the swab entry hole part.

2. The specimen tool according to claim 1, wherein the swab entry hole part is formed on a sight glass corresponding to the mouth/nose of the subject, wherein the sight glass is composed of a transparent material protruding forward from a body of the mask.

3. The specimen tool according to claim 2, wherein the sight glass corresponding to the mouth of the subject is further provided with a tongue pressing part, in which a receiving groove is formed to be spaced apart from the swab entry hole part corresponding to the nose of the subject and to receive a tongue of the subject,
a guide groove is formed between the tongue pressing part and the sigh glass corresponding to the nose of the subject, through which a tongued depresser can be inserted, and
the swab entry hole part corresponding to the mouth of the subject is present on the guide groove, so that an examiner inserts the tongue depresser into the guide groove in order to suppress the tongue of the subject received in the receiving groove.

4. The specimen tool according to any one of claim 1 to 3, wherein the swab entry hole part protrudes from the mask and is formed to have a diameter becoming narrower in a protruded direction, and
the detachable cap is configured in a hollow shape which can be forcedly fitted to the swab entry hole part.

5. The specimen tool according to any one of claims 1 to 3, wherein the swab entry hole part protrudes from the mask, in which a tip of the swab entry hole part is formed to be larger than the diameter of the swab entry hole part, thereby forming a step due to a difference in diameters,
the detachable cap has a hook part to be caught and coupled to the step.

6. The specimen tool according to any one of claims 1 to 3, wherein the detachable cap is made of a resin material having a restoration force, and
the opening means is configured as an incision line provided on a portion of the detachable cap,
wherein, when the specimen collection cotton swab is inserted, the opening means is cut to form an incision by an external force and the incision is rolled into the detachable cap to enable the detachable cap to be kept airtight.

7. The specimen tool according to any one of claims 1 to 3, wherein the specimen collection cotton swab is provided with an interference plate that has a diameter larger than a diameter of the specimen collection cotton swab,
wherein, when the interference plate is inserted into and then exits from the swab entry hole part, the interference plate interferes with the opening means to thus separate the detachable cap.
